# FASCICULE DE BREVET EUROPEEN

(11) **EP 3 846 871 B1**
(45) Date de publication et mention de la délivrance du brevet: **29.06.2022**
(21) Numéro de dépôt: 19758933.6
(22) Date de dépôt: 20.08.2019
(51) Int. Cl.: A61M 60/148, A61M 60/435

(54) **SYSTEME DE GENERATION D'UNE CIRCULATION SANGUINE**
SYSTEM ZUR ERZEUGUNG VON BLUTZIRKULATION
SYSTEN FOR GENERATING BLOOD CIRCULATION

(30) Priorité: 05.09.2018 FR 1857952
(43) Date de publication de la demande: 14.07.2021
(73) Titulaire: Procope Medicals, 44000 Nantes (FR)
(72) Inventeur: CHABANE, Saïd, 44100 NANTES (FR); PLUMEJAULT, Samuel, 49000 ANGERS (FR)
(74) Mandataire: Ermeneux, Bertrand
(86) Numéro de dépôt international: PCT/EP2019/072277
(87) Numéro de publication internationale: WO 2020/048768

(56) Documents cités:
- EP-A1- 0 467 999
- US-A- 3 597 766
- US-A- 3 755 825
- US-A- 4 516 567

## Description

Le domaine de l'invention est celui de la conception et de la fabrication de dispositifs et d'équipements médicaux.

Plus précisément, l'invention concerne un équipement permettant de créer artificiellement un débit sanguin classiquement assuré par le cœur d'un vertébré. L'invention concerne en particulier mais non exclusivement un équipement médical implantable dans la cavité péricardique d'un vertébré.

Dans le domaine de l'invention, on connait les équipements mettant en œuvre le concept de pompe pneumatique et ceux mettant en œuvre le concept de pompe hydraulique.

Historiquement, les premiers cœurs artificiels ont utilisé le concept des pompes pneumatiques. Ces cœurs artificiels doivent être implantés à l'intérieur de la cage thoracique d'un patient, et un équipement externe, apte à générer une pression d'air, est raccordé au cœur artificiel pour l'actionner à l'aide d'un flux d'air.

Dans cette technologie, le cœur artificiel comprend des cavités artificielles à l'intérieur desquelles sont situées des membranes souples. Au sein d'une cavité, une membrane sépare alors de manière étanche une chambre de circulation du sang et une chambre destinée à contenir du gaz. Par l'intermédiaire de tuyaux de raccordement couplant l'équipement externe au cœur artificiel, de l'air comprimé pulsé alimente la chambre destinée à contenir le gaz dans chacune des cavités artificielles pour générer un flux sanguin grâce au principe de la pompe à membrane.

Ce type de solution est robuste et fiable.

Ce concept nécessite toutefois un équipement externe particulièrement lourd et implique l'introduction de canalisations d'air à l'intérieur du corps jusqu'au cœur artificiel. Ainsi, le patient équipé de ce cœur artificiel ne bénéficie pas d'une autonomie importante.

Selon le deuxième type de technologie, il a été proposé un cœur artificiel intégrant un système moteur hydraulique.

Ce cœur hydraulique présente une conception anthropomorphique et permet de gagner une autonomie importante par rapport aux cœurs artificiels précédemment décrits, mettant en œuvre la technologie pneumatique.

En effet, ce type de cœur artificiel hydraulique, reprenant l'idée de la pompe à membrane, intègre le système moteur permettant de générer une pression pulsatile directement à l'intérieur du cœur artificiel.

Un tel cœur artificiel utilise une batterie provisoire implantée à l'intérieur du corps du vertébré, et ne nécessite qu'un système externe de rechargement de la batterie qui n'implique pas de connectique invasive destinées à rentrer à l'intérieur du corps pour alimenter électriquement le cœur.

Un tel système présente néanmoins une robustesse et une fiabilité plus faibles que celles des cœurs mettant en œuvre une technologie pneumatique.

On connaît encore la solution décrite dans le document de brevet publié sous le numéro US4516567, qui divulgue un cœur artificiel pneumatique total fonctionnant avec une seule pompe. La pompe injecte ou aspire de l'air au niveau de membranes pour générer un flux de sang.

Un tel cœur présente comme inconvénient de nécessiter une pompe performante apte à être fortement sollicitée lors de chaque sous-période d'un cycle de fonctionnement cardiaque, la pompe injectant/aspirant directement le volume de fluide déplacé vers les membranes ou à partir de celles-ci.

L'invention a notamment pour objectif de résoudre ces inconvénients de l'état de la technique.

L'invention a notamment pour objectif de proposer un système de génération de circulation sanguine qui soit plus robuste et fiable que les solutions de l'art antérieur mettant en œuvre le concept de cœur hydraulique.

L'invention a également pour objectif de fournir un tel système de génération d'une circulation sanguine qui soit peu consommateur en énergie.

L'invention a aussi pour objectif de fournir un tel système qui soit moins encombrant que les solutions selon l'art antérieur qui mettent en œuvre le concept de pompe pneumatique.

L'invention a plus précisément pour objectif de fournir une telle solution qui soit si peu encombrante qu'elle peut aisément être incorporée à l'intérieur d'un cœur artificiel total destiné à être implanté dans la cage thoracique d'un vertébré.

Ces objectifs, ainsi que d'autres qui apparaitront par la suite, sont atteints grâce à l'invention qui a pour objet un système de génération d'une circulation sanguine dans au moins une partie d'un organe d'un vertébré, comprenant une première cavité artificielle et une deuxième cavité artificielle, lesdites cavités comprenant chacune une membrane souple apte à battre sous l'action d'un gaz, chacune desdites membranes séparant de façon étanche une chambre de circulation du sang et une chambre contenant ledit gaz, caractérisé en ce qu'il comprend :
- un premier réservoir tampon de gaz destiné à être porté sensiblement à une première pression, dite basse pression, et un deuxième réservoir tampon de gaz destiné à être porté sensiblement à une deuxième pression supérieure à ladite première pression, dite haute pression ;
- des moyens de distribution de gaz reliés aux chambres contenant ledit gaz desdites première et deuxième cavités artificielles et auxdits premier et deuxième réservoirs tampon, agencés de sorte à alternativement injecter du gaz dans lesdites chambres contenant ledit gaz et expulser du gaz depuis lesdites chambres contenant ledit gaz pour assurer des valeurs prédéterminées de débits sanguins dans les chambres de circulation du sang de ladite première cavité et de ladite deuxième cavité ;
- une pompe pneumatique alimentée en énergie électrique montée entre ledit premier réservoir et ledit deuxième réservoir tampon et destinée à aspirer du gaz dans ledit premier réservoir pour l'injecter dans ledit deuxième réservoir.

Grâce au système de génération selon l'invention, une circulation sanguine peut être artificiellement générée sans que le système ne soit aussi encombrant que les systèmes pneumatiques selon l'art antérieur employant un équipement externe. Le système de génération selon l'invention peut également présenter une consommation en énergie bien moindre que celle des systèmes pneumatiques selon l'art antérieur.

En effet, selon le principe de l'invention, la pompe pneumatique a pour seul rôle de maintenir une différence de pression entre le premier réservoir tampon de gaz et le deuxième réservoir tampon de gaz.

Cette pompe, à la différence de l'art antérieur, n'a pas pour vocation à directement injecter et aspirer le fluide moteur (ici le gaz) au niveau des chambres contenant ledit gaz de chaque cavité artificielle.

Selon l'invention, il s'agit du premier réservoir tampon de gaz et du deuxième réservoir tampon de gaz qui permettent d'alimenter ou de soutirer du fluide moteur au niveau des chambres contenant ledit gaz pour faire battre les membranes souples et créer une circulation sanguine.

De ce fait, l'utilisation d'une seule pompe pour générer une différence de pression entre le premier réservoir tampon de gaz et le deuxième réservoir tampon de gaz permet au système de présenter une consommation énergétique globale faible, et notamment plus faible que celle des systèmes pneumatiques selon l'art antérieur.

En d'autres termes, le premier réservoir tampon de gaz et le deuxième réservoir tampon de gaz servent de réserve de stockage d'énergie. La distribution de cette énergie est permise et contrôlée par les moyens de distribution de gaz qui relient les chambres contenant ledit gaz au premier réservoir tampon de gaz et au deuxième réservoir tampon de gaz.

Préférentiellement, lesdites première et deuxième cavités artificielles, lesdits premier et deuxième réservoirs tampon de gaz, lesdits moyens de distribution de gaz et ladite pompe pneumatique forment un ensemble monobloc et le système de génération d'une circulation sanguine comprend en outre une batterie d'alimentation en énergie électrique de ladite pompe et desdits moyens de distribution de gaz.

De cette façon, le système de génération selon l'invention peut, au moins temporairement, se suffire à lui-même et présente une autonomie avantageuse pour alimenter en sang le ou les organes raccordés au système.

Selon une caractéristique préférée, ladite pompe est une pompe à palettes.

La pompe à palettes, utilisée pour générer une pression élevée dans le deuxième réservoir tampon de gaz, et une pression plus basse dans le premier réservoir tampon de gaz, est particulièrement adaptée pour permettre au système de génération selon l'invention de présenter une consommation énergétique globale faible.

Cette pompe à palettes compense les fuites internes et les débits consommés par l'actionnement de la pompe cardiaque.

Selon une autre caractéristique avantageuse, lesdits moyens de distribution de gaz comprennent au moins un commutateur piézoélectrique et/ou au moins un commutateur à mémoire de forme et/ou au moins un commutateur électromagnétique.

De tels moyens de distribution de gaz forment des solutions particulièrement adaptées pour miniaturiser ces moyens de distribution.

Préférentiellement, ledit gaz est de l'air.

Un tel gaz permet au système de fonctionner normalement et simplement sans nécessiter un apport d'un gaz spécifique qui serait coûteux et potentiellement difficile à obtenir.

Il convient de noter que les valeurs de la première pression et de la deuxième pression sont ajustées en fonction du patient.

La pression dans les réservoirs tampon est régulée en fonction du rythme cardiaque pour assurer un débit sanguin correspondant aux besoins du patient.

Cet asservissement se fait électroniquement via des capteurs intégrés au système.

Avantageusement, lesdits moyens de distribution de gaz comprennent un distributeur à 4 voies et 2 positions.

Ces moyens de distribution de gaz permettent d'assurer les échanges gazeux entre le premier réservoir tampon de gaz et le deuxième réservoir tampon de gaz avec les chambres contenant ledit gaz des cavités artificielles.

Dans ce cas, de manière préférentielle, ledit distributeur est un distributeur à clapets et/ou à tiroirs piloté.

Selon une première solution préférée, le système de génération d'une circulation sanguine forme une prothèse cardiaque totale destinée à être implantée dans la cavité péricardique d'un patient et apte à remplacer les ventricules gauche et droit dudit patient après ablation de ceux-ci, la première et la deuxième cavité formant un module biventriculaire, la chambre de circulation du sang de la première cavité artificielle étant destinée à être reliée à l'oreillette gauche et à l'aorte dudit patient et la chambre de circulation du sang de la deuxième cavité artificielle étant destinée à être reliée à l'oreillette droite et à l'artère pulmonaire dudit patient.

Une telle prothèse cardiaque totale permet alors d'allier la robustesse et la fiabilité des implants pneumatiques au faible encombrement des prothèses totales utilisant un fluide moteur hydraulique selon l'art antérieur. De plus, la prothèse cardiaque totale du système de génération total selon l'invention peut être complètement autonome et ne nécessiter qu'un faible apport d'électricité pour faire fonctionner les moyens de distribution de gaz et la pompe pneumatique.

Selon une deuxième solution préférentielle, le système de génération d'une circulation sanguine forme un circuit de perfusion ex-vivo dudit organe, permettant de maintenir ledit organe en vie pour une greffe.

En effet, le système de génération selon l'invention permet alors d'assurer une circulation sanguine extracorporelle grâce à un appareillage qui est particulièrement compact, qui est moins encombrant que ceux connus dans l'art antérieur, et qui ne nécessite qu'un très faible apport énergétique (de l'ordre de 15W à 20W).

De préférence, un système de génération d'une circulation sanguine telle que ceux décrits ci-dessus comprend un et une seule pompe pneumatique.

D'autres caractéristiques et avantages de l'invention apparaîtront plus clairement à la lecture de la description suivante d'un mode de réalisation préférentiel de l'invention, donné à titre d'exemple illustratif et non limitatif, et des dessins annexés parmi lesquels :
- la figure 1 est une représentation schématique du système de génération d'une circulation sanguine selon l'invention, dans lequel les chambres contenant ledit gaz sont reliées au deuxième réservoir tampon de gaz (haute pression), les chambres de circulation du sang présentant alors un volume faible ;
- la figure 2 est une représentation schématique du même système de génération d'une circulation sanguine, dans lequel les chambres contenant ledit gaz sont reliées au premier réservoir tampon de gaz (basse pression), les chambres de circulation du sang présentant alors un volume important.

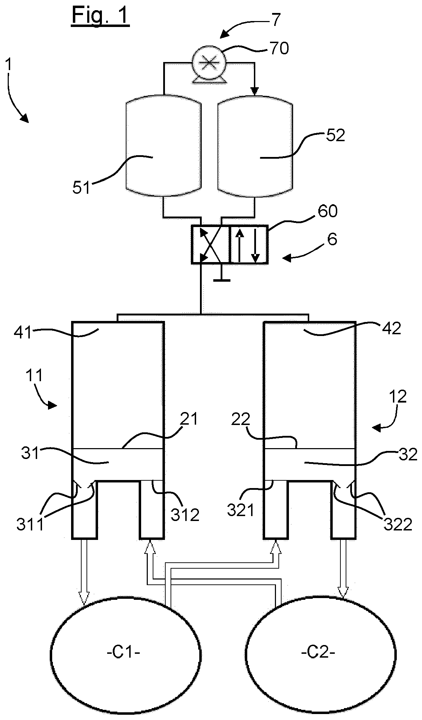

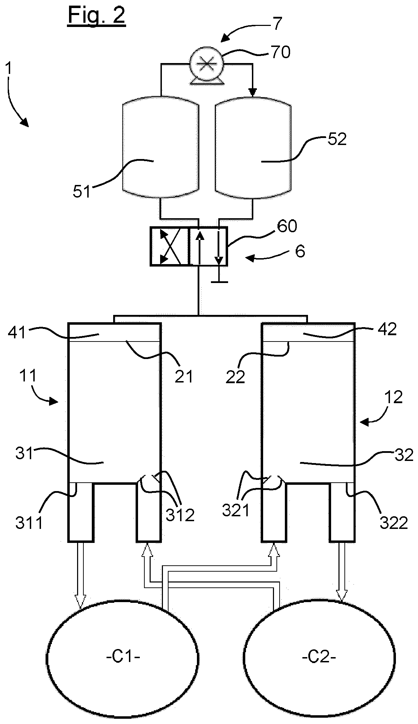

En référence aux figures 1 et 2, le système 1 de génération d'une circulation sanguine permet de générer artificiellement un flux sanguin dans au moins une partie d'un organe d'un vertébré. Plus précisément, le système 1 permet de générer un flux sanguin dans deux circuits de circulation d'un flux sanguin, dont :
- un premier circuit de circulation du flux sanguin C1 ;
- un deuxième circuit de circulation du flux sanguin C2.

Tel que cela sera détaillé par la suite, le système 1 de génération d'une circulation sanguine peut former selon une première solution préférentielle une prothèse cardiaque totale. Dans ce cas, par exemple, le premier circuit de circulation du flux sanguin C1 correspond à un circuit de circulation anatomique alimentant le corps d'un vertébré en sang oxygéné, et le deuxième circuit de circulation du flux sanguin C2 correspond à un autre circuit de circulation anatomique destiné à oxygéner le sang (cet autre circuit de circulation intégrant notamment les poumons du vertébré).

Selon une deuxième solution préférentielle, le système 1 de génération d'une circulation sanguine peut former un circuit de circulation sanguine ex-vivo d'un organe. Dans ce cas, au moins l'un du premier circuit de circulation du flux sanguin C1 ou du deuxième circuit de circulation du flux sanguin C2 est alors couplé à l'organe pour l'alimenter en sang.

Tel qu'illustré par les figures 1 et 2, le système 1 de génération d'une circulation sanguine comprend :
- une première cavité 11 artificielle ;
- une deuxième cavité 12 artificielle ;
- un premier réservoir tampon 51 de gaz ;
- un deuxième réservoir tampon 52 de gaz ;
- des moyens de distribution de gaz 6 couplant la première cavité 11 artificielle et la deuxième cavité 12 artificielle au premier réservoir tampon 51 de gaz et au deuxième réservoir tampon 52 de gaz ;
- une pompe 7 pneumatique couplée au premier réservoir tampon 51 de gaz et au deuxième réservoir tampon 52 de gaz.

La première cavité 11 artificielle et la deuxième cavité 12 artificielle comprennent chacune une membrane 21, 22 souple apte à battre sous l'action d'un gaz. En effet, le système 1 de génération d'une circulation sanguine est un système pneumatique et utilise un gaz comme fluide moteur pour permettre la génération de la circulation sanguine. Ce gaz est préférentiellement de l'air.

Ces membranes 21, 22 souples sont élastiques et divisent, respectivement, la première cavité 11 artificielle et de la deuxième cavité 12 artificielle en deux chambres.

Plus précisément :
- la membrane 21 souple de la première cavité 11 artificielle sépare de façon étanche, au sein de cette première cavité 11, une chambre de circulation du sang 31 et une chambre contenant ledit gaz 41 ;
- la membrane 22 souple de la deuxième cavité 12 artificielle sépare de façon étanche, au sein de cette deuxième cavité 12, une chambre de circulation du sang 32 et une chambre contenant ledit gaz 42.

La première cavité 11 artificielle et la deuxième cavité 12 artificielle comprennent également :
- des clapets d'injection 312, 321 du sang dans les chambres de circulation du sang 31, 32 ;
- des clapets d'éjection 311, 322 du sang depuis les chambres de circulation du sang 31, 32.

Ces clapets d'injection 312, 321 et ces clapets d'éjection 311, 322 permettent de garantir le sens de circulation du sang dans le système. Des tarages spécifiques pour chaque clapet 311, 312, 321, 322 permettent de régler les pressions de fonctionnement des cavités artificielles.

Tel qu'expliqué précédemment, les membranes 21, 22 souples sont aptes à battre sous l'action du gaz.

En battant, les membranes 21, 22 souples font chacune varier le volume des chambres de circulation du sang 31, 32.

Lors de l'augmentation du volume d'une chambre de circulation du sang 31, 32, la pression au sein de ladite chambre de circulation du sang 31, 32 chute et provoque la fermeture des clapets d'éjection 311, 322 ainsi que l'ouverture des clapets d'injection 312, 321, permettant de remplir de sang la chambre de circulation du sang 31, 32.

Au contraire, lors de la diminution du volume d'une chambre de circulation du sang 31, 32, la pression au sein de ladite chambre de circulation du sang 31, 32 augmente et provoque la fermeture des clapets d'injection 312, 321 ainsi que l'ouverture des clapets d'éjection 311, 322, permettant d'expulser le sang hors de la chambre de circulation du sang.

Les échanges gazeux (injection et extraction) au niveau des chambres contenant ledit gaz 41, 42 sont assurés par :
- les moyens de distribution de gaz 6 ;
- le premier réservoir tampon 51 de gaz ;
- le deuxième réservoir tampon 52 de gaz ;
- la pompe 7 pneumatique.

Le premier réservoir tampon 51 de gaz est destiné à être porté sensiblement à une première pression, dite basse pression.

Quant à lui, le deuxième réservoir tampon 52 de gaz est destiné à être porté sensiblement à une deuxième pression, dite haute pression, la deuxième pression étant supérieure à la première pression.

A titre d'exemple, la première pression peut être portée à 1,1 bar et la deuxième pression peut être portée à 1,15 bar.

De préférence, le fonctionnement de la pompe pneumatique est dépendant du « rythme cardiaque » défini par les moyens de distribution de gaz, pour garder les pressions dans les limites ci-dessus.

Dans ce cas, l'asservissement du système 1 est réalisé électroniquement par l'intermédiaire de capteurs intégrés au système.

Comme illustré sur les figures 1 et 2, la pompe 7 pneumatique est montée entre le premier réservoir tampon 51 et le deuxième réservoir tampon 52.

La pompe 7 pneumatique aspire du gaz dans le premier réservoir tampon 51 pour l'injecter dans le deuxième réservoir tampon 52. De cette manière, la pompe 7 pneumatique permet le maintien du premier réservoir tampon 51 sensiblement à sa basse pression et le maintien du deuxième réservoir tampon 52 sensiblement à sa haute pression.

Pour fonctionner, cette pompe pneumatique 7 est alimentée en énergie électrique.

Cette pompe est avantageusement une pompe à palettes 70. La pompe à palettes est une pompe de transfert volumétrique, constituée par un stator (fixe) et un rotor (mobile) qui tourne tangentiellement au stator. Solidaires du rotor, les palettes peuvent coulisser dans des logements du rotor, perpendiculairement à l'axe de rotation du rotor, pour venir en contact des parois du stator par la force centrifuge. En complément de la force centrifuge, le rotor peut éventuellement comporter des moyens de rappel en position des palettes dans une position en contact avec les parois du stator.

De préférence et tel qu'illustré sur le figures, la pompe pneumatique 7 est unique.

Tel qu'expliqué précédemment, les moyens de distribution de gaz 6 sont couplés à la première cavité 11 artificielle, à la deuxième cavité 12 artificielle, ainsi qu'aux réservoirs tampon. Plus précisément, les moyens de distribution de gaz 6 sont raccordés aux chambres contenant ledit gaz 41, 42, au premier réservoir tampon 41, et au deuxième réservoir tampon 42.

Ces moyens de distribution de gaz 6 sont agencés de sorte à alternativement injecter du gaz dans les chambres contenant ledit gaz 41, 42 et extraire du gaz depuis les chambres contenant ledit gaz 41, 42, pour assurer des valeurs prédéterminées de débits sanguins dans les chambres de circulation du sang des cavités artificielles.

En d'autres termes, les moyens de distribution de gaz 6 (ainsi que les réservoirs tampon) permettent de modifier la pression des chambres contenant ledit gaz 41, 42 de manière à provoquer une expansion ou une diminution du volume de ces chambres grâce à la déformation élastique des membranes 21, 22 souples.

Une variation répétitive du volume des chambres contenant ledit gaz entraîne mécaniquement une variation répétitive du volume des chambres de circulation du sang 31, 32.

Cette variation du volume des chambres de circulation du sang 31, 32 se traduit également par une variation de la pression du sang au sein de ces chambres.

De ce fait, les moyens de distribution de gaz 6, en injectant et en extrayant du gaz de manière alternative, permettent de créer un flux sanguin, en pompant le sang à l'intérieur des chambres de circulation du sang 31, 32 et en expulsant le sang depuis ces chambres de circulation du sang 31, 32 grâce aux clapets d'injection 312, 321 et aux clapets d'éjection 311, 322 précédemment décrits.

Préférentiellement, les moyens de distribution de gaz 6 comprennent au moins un commutateur piézoélectrique et/ou au moins un commutateur à mémoire de forme et/ou au moins un commutateur électromagnétique.

Tel qu'illustré par les figures 1 et 2, les moyens de distribution de gaz 6 comprennent un distributeur 60 à 4 voies et 2 positions. Le distributeur 60 peut être un distributeur 60 à tiroirs piloté, ou encore un distributeur à clapets.

En effet, en référence à la figure 1, le distributeur 60 adopte une première position dans laquelle le deuxième réservoir tampon 52 (haute pression) communique avec la chambre contenant ledit gaz 41 de la première cavité 11 artificielle ainsi qu'avec la chambre contenant ledit gaz 42 de la deuxième cavité 12 artificielle. Dans ce cas, le gaz sous haute pression situé dans le deuxième réservoir tampon 52 est injecté dans les chambres contenant ledit gaz 41, 42.

Suite au passage du distributeur 60 dans sa deuxième position et tel qu'illustré par la figure 2, le deuxième réservoir tampon 52 ne communique plus avec les chambres contenant ledit gaz 41, 42. En effet, ces chambres contenant ledit gaz 41, 42 communiquent alors avec le premier réservoir tampon 51 (basse pression) et le gaz.

Selon le présent mode de réalisation, les chambres contenant ledit gaz 41, 42 communiquent en même temps avec le premier réservoir tampon 51 ou avec le deuxième réservoir tampon 52. En conséquence, les chambres de circulation du sang 31, 32 se remplissent et expulsent le sang de manière synchronisée.

Selon d'autres modes de réalisation envisageables, le distributeur peut être configuré de manière à désynchroniser le remplissage et l'expulsion du sang des chambres de circulation du sang 31, 32 et/ou à réaliser ces remplissages ou expulsion à un rythme différent entre chaque cavité artificielle.

Préférentiellement, les première 11 et deuxième 12 cavités artificielles, les premier 51 et deuxième 52 réservoirs tampon de gaz, les moyens de distribution de gaz 6 et la pompe pneumatique 7 forment un ensemble monobloc.

Le système 1 de génération d'une circulation sanguine comprend par ailleurs une batterie d'alimentation en énergie électrique de la pompe 7 pneumatique et des moyens de distribution de gaz 6 destinée à être montée dans une cavité abdominale. Le rechargement de cette batterie s'effectue par induction transcutanée.

Tel qu'évoqué précédemment, le système 1 de génération d'une circulation sanguine peut former une prothèse cardiaque totale. Dans ce cas, le système 1 est destiné à être implanté dans la cavité péricardique d'un patient. Le système 1 permet alors de remplacer les ventricules (gauche et droit) du patient. La première cavité 11 artificielle et la deuxième cavité 12 artificielle forment ainsi un module biventriculaire, la chambre de circulation du sang 31 de la première cavité 11 artificielle étant alors reliée à l'oreillette gauche et à l'aorte du patient, et la chambre de circulation du sang 32 de la deuxième cavité 12 artificielle étant alors reliée à l'oreillette droite et à l'artère pulmonaire du patient.

Un exemple de déroulement du cycle de fonctionnement de la prothèse cardiaque totale est développé ci-après.

La systole correspond à l'éjection du sang contenu des chambres de circulation du sang (ventricules artificiels) vers les circuits de circulation du sang C1, C2.

La diastole correspond à l'injection du sang contenu dans les circuits de circulation du sang C1, C2 (sang en provenance des oreillettes gauche et droite) à l'intérieur des chambres de circulation du sang 31, 32 (aspiration du sang à l'intérieur des ventricules artificiels).

Le passage de la diastole à la systole et inversement se traduit par une modification de l'évolution des pressions au sein des chambres de circulation du sang 31, 32 (augmentation et diminution des pressions) et se traduisent par le changement d'état des clapets d'injection 312, 321 et des clapets d'éjection 311, 322.

Lors du passage de la diastole à la systole, les clapets d'éjection 311, 322 (clapets aortiques et pulmonaires) passent de l'état fermé à l'état ouvert, et les clapets d'injection 312, 321 (clapets auriculo-ventriculaires) passent de l'état ouvert à l'état fermé.

Lors du passage de la systole à la diastole, les clapets d'éjection 311, 322 (clapets aortiques et pulmonaires) passent de l'état ouvert à l'état fermé, et les clapets d'injection 312, 321 (clapets auriculo-ventriculaires) passent de l'état fermé à l'état ouvert.

A l'état initial de la systole, les chambres contenant ledit gaz 41, 42 présentent chacune un volume de l'ordre de 5 mL avec une pression de l'ordre de 0,107 Bar, les chambres de circulation du sang 31, 32 (ventricules artificiels) sont remplies de sang, le distributeur 60 a été basculé et les chambres contenant ledit gaz 41, 42 viennent d'être connectées au deuxième réservoir tampon 52 (haute pression).

La pression dans les chambres contenant ledit gaz 41, 42 augmente et provoque la déformation des membranes 21, 22 élastiques. La pression dans les chambres contenant ledit gaz augmente notamment jusqu'à 0,160 Bar.

Les chambres de circulation du sang 31, 32 se vident et le sang est envoyé vers les circuits de circulation du sang C1, C2 (organes et les poumons).

A l'état initial de la diastole, les chambres contenant ledit gaz présentent chacune un volume de l'ordre de 130 mL avec une pression de l'ordre de 0,160 Bar, les chambres de circulation du sang 31, 32 (ventricules artificiels) sont vides de sang (ou à leur niveau le plus faible), le distributeur a été basculé et les chambres contenant ledit gaz 41, 42, viennent d'être connectées au premier réservoir tampon 51 (basse pression).

La pression dans les chambres contenant ledit gaz 41, 42 chute (en revenant alors à 0,107 Bar) et provoque la déformation des membranes 21, 22 élastique entrainant l'augmentation du volume des chambres de circulation du sang 31, 32.

Les chambres de circulation du sang 31, 32 (ventricules artificiels) se remplissent.

Le système 1 selon l'invention est configuré pour que les chambres de circulation du sang (ventricules artificiels) se remplissent à pression constante, soit en moyenne :
- 0,012 Bar pour la chambre de circulation du sang de la première cavité 11 artificielle (ventricule artificiel gauche) ;
- 0,005 Bar pour la chambre de circulation du sang de la deuxième cavité 12 artificielle (ventricule artificiel droit).

A la fin de la diastole, le cycle systole-diastole peut alors recommencer.

## Revendications

1. Système (1) de génération d'une circulation sanguine dans au moins une partie d'un organe d'un vertébré, comprenant une première cavité (11) artificielle et une deuxième cavité (12) artificielle, lesdites cavités comprenant chacune une membrane (21), (22) souple apte à battre sous l'action d'un gaz, chacune desdites membranes séparant de façon étanche une chambre de circulation du sang (31), (32) et une chambre contenant ledit gaz (41), (42),
**caractérisé en ce qu'**il comprend :
- un premier réservoir tampon (51) de gaz destiné à être porté sensiblement à une première pression, dite basse pression, et un deuxième réservoir tampon (52) de gaz destiné à être porté sensiblement à une deuxième pression supérieure à ladite première pression, dite haute pression ;
- des moyens de distribution de gaz (6) reliés aux chambres contenant ledit gaz desdites première et deuxième cavités artificielles et auxdits premier et deuxième réservoirs tampon, agencés de sorte à alternativement injecter du gaz dans lesdites chambres contenant ledit gaz et expulser du gaz depuis lesdites chambres contenant ledit gaz pour assurer des valeurs prédéterminées de débits sanguins dans les chambres de circulation du sang de ladite première cavité et de ladite deuxième cavité ;
- une pompe (7) pneumatique alimentée en énergie électrique montée entre ledit premier réservoir tampon et ledit deuxième réservoir tampon et destinée à aspirer du gaz dans ledit premier réservoir pour l'injecter dans ledit deuxième réservoir.

2. Système (1) de génération d'une circulation sanguine selon la revendication 1, **caractérisé en ce que** lesdites première et deuxième cavités artificielles, lesdits premier et deuxième réservoirs tampon de gaz, lesdits moyens de distribution de gaz et ladite pompe pneumatique forment un ensemble monobloc et **en ce qu'**il comprend en outre une batterie d'alimentation en énergie électrique de ladite pompe (7) et desdits moyens de distribution de gaz (6).

3. Système (1) de génération d'une circulation sanguine selon l'une quelconque des revendications 1 et 2, **caractérisé en ce que** ladite pompe (7) est une pompe à palettes (70).

4. Système (1) de génération d'une circulation sanguine selon l'une quelconque des revendications 1 à 3, **caractérisé en ce que** lesdits moyens de distribution de gaz (6) comprennent au moins un commutateur piézoélectrique et/ou au moins un commutateur à mémoire de forme et/ou au moins un commutateur électromagnétique.

5. Système (1) de génération d'une circulation sanguine selon l'une quelconque des revendications 1 à 4, **caractérisé en ce que** ledit gaz est de l'air.

6. Système (1) de génération d'une circulation sanguine selon l'une quelconque des revendications 1 à 5, **caractérisé en ce que** lesdits moyens de distribution de gaz (6) comprennent un distributeur (60) à 4 voies et 2 positions.

7. Système (1) de génération d'une circulation sanguine selon la revendication 6, **caractérisé en ce que** ledit distributeur (60) est un distributeur à clapets et/ou à tiroirs piloté.

8. Système (1) de génération d'une circulation sanguine selon l'une quelconque des revendications 1 à 7, **caractérisé en ce qu'**il forme une prothèse cardiaque totale destinée à être implantée dans la cavité péricardique d'un patient et apte à remplacer les ventricules gauche et droit dudit patient après ablation de ceux-ci, la première et la deuxième cavité (11), (12) formant un module biventriculaire, la chambre de circulation du sang (31) de la première cavité (11) artificielle étant destinée à être reliée à l'oreillette gauche et à l'aorte dudit patient et la chambre de circulation du sang (32) de la deuxième cavité (12) artificielle étant destinée à être reliée à l'oreillette droite et à l'artère pulmonaire dudit patient.

9. Système (1) de génération d'une circulation sanguine selon l'une quelconque des revendications 1 à 7, **caractérisé en ce qu'**il forme un circuit de perfusion ex-vivo dudit organe, permettant de maintenir ledit organe en vie pour une greffe.

10. Système (1) de génération d'une circulation sanguine selon l'une quelconque des revendications 1 à 9, **caractérisé en ce qu'**il comprend un et une seule pompe (7) pneumatique.

## Patentansprüche

1. System (1) zum Erzeugen einer Blutzirkulation in mindestens einem Teil eines Organs eines Wirbeltiers, umfassend einen ersten künstlichen Hohlraum (11) und einen zweiten künstlichen Hohlraum (12), wobei die Hohlräume jeweils eine elastische Membran (21), (22) umfassen, die imstande ist, unter der Wirkung eines Gases zu schlagen, wobei jede der Membranen eine Blutzirkulationskammer (31), (32) und eine Kammer, die das Gas enthält (41), (42), dicht trennt,
**dadurch gekennzeichnet, dass** es umfasst:
- einen ersten Gaspufferspeicher (51), der dazu bestimmt ist, auf etwa einen ersten Druck, bezeichnet als niedriger Druck, gebracht zu werden, und einen zweiten Gaspufferspeicher (52), der dazu bestimmt ist, auf etwa einen zweiten Druck gebracht zu werden, der höher als der erste Druck ist, bezeichnet als hoher Druck;
- Gasverteilermittel (6), die mit den Kammern verbunden sind, die das Gas des ersten und zweiten künstlichen Hohlraums enthalten und mit dem ersten und zweiten Pufferspeicher, die derart eingerichtet sind, dass abwechselnd Gas in die Kammern eingeleitet wird, die das Gas enthalten, und Gas aus den Kammern ausgeleitet wird, die das Gas enthalten, um vorher festgelegte Blutdurchflusswerte in den Blutzirkulationskammern des ersten Hohlraums und des zweiten Hohlraums sicherzustellen;
- eine pneumatische Pumpe (7), die mit elektrischer Energie versorgt wird, die zwischen dem ersten Pufferspeicher und dem zweiten Pufferspeicher angebracht ist und bestimmt, Gas im ersten Vorratsbehälter anzusaugen, um es in den zweiten Vorratsbehälter einzuleiten.

2. System (1) zum Erzeugen einer Blutzirkulation nach Anspruch 1, **dadurch gekennzeichnet, dass** der erste und zweite künstliche Hohlraum, der erste und zweite Gaspufferspeicher, die Gasverteilermittel und die pneumatische Pumpe eine einteilige Einheit bilden und dass es ferner eine Batterie zur Versorgung der Pumpe (7) und der Gasverteilermittel (6) mit elektrischer Energie umfasst.

3. System (1) zum Erzeugen einer Blutzirkulation nach einem der Ansprüche 1 und 2, **dadurch gekennzeichnet, dass** die Pumpe (7) eine Flügelpumpe (70) ist.

4. System (1) zum Erzeugen einer Blutzirkulation nach einem der Ansprüche 1 bis 3, **dadurch gekennzeichnet, dass** die Gasverteilermittel (6) mindestens einen piezoelektrischen Schalter und/oder mindestens einen Schalter mit Formengedächtnis und/oder mindestens einen elektromagnetischen Schalter umfassen.

5. System (1) zum Erzeugen einer Blutzirkulation nach einem der Ansprüche 1 bis 4, **dadurch gekennzeichnet, dass** das Gas Luft ist.

6. System (1) zum Erzeugen einer Blutzirkulation nach einem der Ansprüche 1 bis 5, **dadurch gekennzeichnet, dass** die Gasverteilermittel (6) einen Verteiler (60) mit 4 Wegen und 2 Positionen umfassen.

7. System (1) zum Erzeugen einer Blutzirkulation nach Anspruch 6, **dadurch gekennzeichnet, dass** der Verteiler (60) ein Verteiler mit Ventilen und/oder und oder gesteuert mit Schiebern ist.

8. System (1) zum Erzeugen einer Blutzirkulation nach einem der Ansprüche 1 bis 7, **dadurch gekennzeichnet, dass** es eine vollständige Herzprothese bildet, die zur Implantation in die Perikardhöhle eines Patienten bestimmt ist und imstande, den linken und rechten Ventrikel des Patienten nach Ablation derselben zu ersetzen, wobei der erste und der zweite Hohlraum (11), (12) ein biventrikuläres Modul bilden, wobei die Blutzirkulationskammer (31) des ersten künstlichen Hohlraums (11) bestimmt ist, mit dem linken Vorhof und mit der Aorta des Patienten verbunden zu sein und die Blutzirkulationskammer (32) des zweiten künstlichen Hohlraums (12) bestimmt ist, mit dem rechten Vorhof und mit der Lungenarterie des Patienten verbunden zu sein.

9. System (1) zum Erzeugen einer Blutzirkulation nach einem der Ansprüche 1 bis 7, **dadurch gekennzeichnet, dass** es einen ex-vivo-Perfusionskreis des Organs bildet, der erlaubt, das Organ für eine Spende am Leben zu erhalten.

10. System (1) zum Erzeugen einer Blutzirkulation nach einem der Ansprüche 1 bis 9, **dadurch gekennzeichnet, dass** es eine und eine einzige pneumatische Pumpe (7) umfasst.

## Claims

1. A system (1) for generating a blood circulation in at least part of an organ of a vertebrate, comprising a first artificial cavity (11) and a second artificial cavity (12), said cavities each comprising a flexible membrane (21), (22) capable of beating under the action of a gas, each of said membranes sealingly separating a blood circulation chamber (31), (32) and a chamber containing said gas (41), (42),
**characterised in that** it comprises
- a first gas buffer reservoir (51) intended to be brought substantially to a first pressure, referred to as low pressure, and a second gas buffer reservoir (52) intended to be brought substantially to a second pressure higher than said first pressure, referred to as high pressure;
- gas distribution means (6) connected to the chambers containing said gas of said first and second artificial cavities and to said first and second buffer reservoirs, arranged to alternately inject gas into said chambers containing said gas and expel gas from said chambers containing said gas to provide predetermined values of blood flow rates in the blood circulation chambers of said first cavity and said second cavity;
- a pneumatic pump (7) supplied with electrical energy and mounted between said first buffer reservoir and said second buffer reservoir and intended to suck gas from said first reservoir in order to inject it into said second reservoir.

2. The system (1) for generating a blood circulation according to claim 1, **characterised in that** said first and second artificial cavities, said first and second gas buffer reservoirs, said gas distribution means and said pneumatic pump form a single-piece assembly and **in that** it further comprises a battery for supplying electrical energy to said pump (7) and said gas distribution means (6).

3. The system (1) for generating a blood circulation according to any one of claims 1 and 2, **characterised in that** said pump (7) is a vane pump (70).

4. The system (1) for generating a blood circulation according to any one of claims 1 to 3, **characterised in that** said gas distribution means (6) comprise at least one piezoelectric switch and/or at least one shape memory switch and/or at least one electromagnetic switch.

5. The system (1) for generating a blood circulation according to any one of claims 1 to 4, **characterised in that** said gas is air.

6. The system (1) for generating a blood circulation according to any one of claims 1 to 5, **characterised in that** said gas distribution means (6) comprises a 4-way, 2-position valve (60).

7. The system (1) for generating a blood circulation according to claim 6, **characterised in that** said valve (60) is a flap valve and/or a pilot operated slide valve.

8. The system (1) for generating a blood circulation according to any of claims 1 to 7, **characterised in that** it forms a total cardiac prosthesis intended to be implanted in the pericardial cavity of a patient and capable of replacing the left and right ventricles of said patient after ablation thereof, the first and second cavity (11), (12) forming a biventricular module, the blood circulation chamber (31) of the first artificial cavity (11) being intended to be connected to the left atrium and the aorta of said patient and the blood circulation chamber (32) of the second artificial cavity (12) being intended to be connected to the right atrium and the pulmonary artery of said patient.

9. The system (1) for generating a blood circulation according to any one of claims 1 to 7, **characterised in that** it forms a circuit for the ex-vivo perfusion of said organ, making it possible to keep said organ alive for transplantation.

10. The system (1) for generating a blood circulation according to any one of claims 1 to 9, **characterised in that** it comprises one and only one pneumatic pump (7).
